# EUROPEAN PATENT APPLICATION

(11) **EP 1 604 965 A1**
(43) Date of publication of application: **14.12.2005**
(21) Application number: 04721320.2
(22) Date of filing: 17.03.2004
(51) Int. Cl.: C07C 7/20, C07C 15/46

(54) **POLYMERIZATION INHIBITOR FOR AROMATIC VINYL COMPOUNDS AND METHOD FOR INHIBITING THE POLYMERIZATION OF THE COMPOUNDS**

(30) Priority: 17.03.2003 JP 2003071692
(71) Applicant: Hakuto Co., Ltd, Shinjuku-ku, Tokyo 160-8910 (JP)
(72) Inventor: Nakajima, J., c/o Yokkaichi Lab., Hakuto Co., Ltd., Yokkaichi-shi, Mie 5100007 (JP); Tanizaki, S., c/o Yokkaichi Lab., Hakuto Co., Ltd., Yokkaichi-shi, Mie5100007 (JP)
(74) Representative: Albrecht, Thomas
(86) International application number: PCT/JP2004/003540
(87) International publication number: WO 2004/083156

(57) **Abstract**

According to the present invention, there are provided a polymerization inhibitor which is easy to handle and which has a little toxicity and a high polymerization-inhibitory effect toward an aromatic vinyl compound and a process for inhibiting the polymerization of the aromatic vinyl compound.

The present invention relates to a polymerization inhibitor for the aromatic vinyl compound which comprises contain two ingredients of a specific quinone methide compound and a specific organic sulfonic acid compound, and to a process for inhibiting polymerization of the aromatic vinyl compound which comprises adding a specific quinone methide compound and a specific organic sulfonic acid compound simultaneously or further a specific quinone methide compound, a specific organic sulfonic acid compound and a specific N-oxyl compound simultaneously in the stage of producing, purifying, storing or transporting the aromatic vinyl compound.

## Description

### Technical Field

The present invention relates to a polymerization inhibitor for an aromatic vinyl compound which is effective to prevent the polymerization of an aromatic vinyl compound that occurs during the stage of producing, purifying, storing or transporting the aromatic vinyl compound and which is effective to prevent the occurrence of fouling resulting from the polymerization in a petroleum refinery plant, etc., and to a process for inhibiting the polymerization of the aromatic vinyl compound.

### Background Art

Among aromatic vinyl compounds, especially styrene is an industrially very important compound as raw material for preparing polystyrene, synthetic rubber, ABS resin, etc. and has been industrially manufactured in a large amount.

In general, an aromatic vinyl compound is extremely susceptible to polymerization so that it undergoes easily polymerization under an influence of the heat generated in its preparation or purification stage or during its storage, thereby causing lowering in the yield of the monomeric aromatic vinyl compound and further the formation of soil (fouling) in the facilities involved to induce trouble in operating the facilities. Also, in addition to the preparation and purification stages of aromatic vinyl compound, there is a problem of forming a fouling in a petroleum refinery plant, etc. which originates in the polymerization of aromatic vinyl compound present therein.

As the countermeasure taken hitherto, a process wherein certain polymerization inhibitor is added to process fluid containing an aromatic vinyl compound has been proposed and practically carried out. Specifically, nitrophenols which are represented by 2,4-dinitrophenol have been used. Since nitrophenols have an effect to decrease the polymerization velocity so that they have property capable of inhibiting the polymerization for a relatively long time, they have been widely applied. However, nitrophenols are classified into self-reacting material in the fifth class of the Fire Services Act (Japan) and belong to toxic substance and therefore strict care is required in handling them.

So, as a process to be replaced by one for inhibiting polymerization of an aromatic vinyl compound using these polymerization inhibitors, there has been proposed one wherein a N-oxyl compound is used as thermal polymerization inhibitor of styrene (see JP 1-165534 A). Although the N-oxyl compound is extremely effective for inhibiting the early polymerization of an aromatic vinyl compound, in the case that it itself was completely consumed by reaction with a radical, the polymerization occurs and proceeds at the same velocity as in the absence of the chemical reagent. There was a problem that the inhibition of polymerization can not be expected because N-oxyl compound is completely consumed especially at the bottom of distillation column where the separation and purification of an aromatic vinyl compound is intended and where the residence time of process liquid is relatively long or otherwise at the distillation column positioned following the part to which said polymerization inhibitor is added in the separation process equipping with a plurality of distillation columns.

Also, there was proposed a process wherein N-oxyl compound is used together with nitrophenols in order to prevent early polymerization of aromatic vinyl compound and inhibit the polymerization over a relatively long period of time thereby causing the reduction in the amount used of nitrophenols (see Japanese Patent No. 2855399 and JP 2000-511545 A). However, this process is the same in point with using nitrophenols which are troublesome to handle and therefore it did not become fundamental solution.

On the other hand, there has been proposed a process wherein as compound which does not belong to toxic substance and which is replaceable by nitrophenols, a 7-arylquinone methide is used with or without N-oxyl compound (see JP 9-165408). Also, there has been proposed a process for inhibiting polymerization of an aromatic vinyl compound by using a 7-arylquinone methide in combination with a substituted hydroxylamine (see U.S. Patent No. 6,024,894 specification). Although 7-arylquinone methide is an excellent polymerization inhibitor in safety and handling as compared with dinitrophenols, its polymerization-inhibiting effect is inferior to dinitrophenols. Accordingly a polymerization inhibitor having an excellent inhibitory effect has been strongly desired.

### Disclosure of the Invention

An object of the present invention is to provide a polymerization inhibitor for an aromatic vinyl compound which is easy to handle and which has a little toxicity and a high polymerization inhibitory effect, and further object of the present invention is to provide a process for inhibiting polymerization of an aromatic vinyl compound.

As a result of having studied about the polymerization reaction of an aromatic vinyl compound in detail, the present inventors have found that by employing specific quinone methide together with specific organic sulfonic acid compound the early polymerization preventing term becomes long so that the polymerization may be inhibited over a long period of time, and have completed the present invention.

That is, the invention involving in claim 1 is a polymerization inhibitor for an aromatic vinyl compound which is characterized by containing a quinone methide compound represented by the following general formula (1) and an organic sulfonic acid compound represented by the following general formula (2) 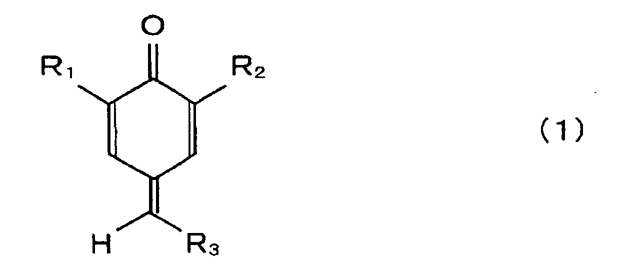 wherein R₁ and R₂ represent independently an alkyl group having 4-18 carbon atoms, a cycloalkyl group having 5-12 carbon atoms or a phenylalkyl group having 7-15 carbon atoms, and R₃ represents a phenyl group or a phenyl group having 1 to 3 substituent groups, said substituent group(s) being selected from an alkyl group having 1-8 carbon atoms, an alkoxyl group having 1-8 carbon atoms, an alkylthio group having 1-8 carbon atoms, an alkoxycarbonyl group having 2-8 carbon atoms, a hydroxyl group, a nitro group, a cyano group, a carboxyl group, a carbamoyl group and chlorine atom. 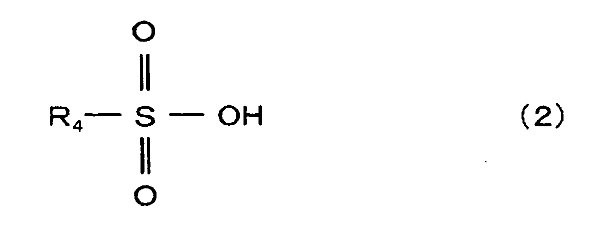 wherein R₄ represents a straight or branched chain alkyl group having 1-32 carbon atoms, an alkylphenyl group wherein the alkyl group is a straight or branched chain one having 1-32 carbon atoms or an alkylnaphthyl group wherein the alkyl group is a straight or branched chain one having 1-32 carbon atoms.

The invention involving in claim 2 is the polymerization inhibitor for an aromatic vinyl compound as claimed in claim 1 wherein said quinone methide compound is one or more members selected from 2,6-di-tert-butyl-4-benzylidene-cyclohexa-2, 5-dienone, 2,6-di-tert-butyl-4-(4-nitrobenzylidene)-cyclohexa-2,5-dienone, 2,6-di-tert-butyl-3-(4-nitrobenzylidene)-cyclohexa-2,5-dienone, 2,6-di-tert-butyl-4-(4-cyanobenzylidene)-cyclohexa-2,5-dienone, 2,6-di-tert-butyl-4-(4-methoxybenzylidene)-cyclohexa-2,5-dienone and 2,6-di-tert-butyl-4-(3,5-di-tert-butyl-4-hydroxybenzylidene)-cyclohexa-2,5-dienone.

The invention involving in claim 3 is the polymerization inhibitor for an aromatic vinyl compound as claimed in claim 1 wherein said organic sulfonic acid compound is one or more members selected from methanesulfonic acid, toluenesulfonic acid, xylenesulfonic acid, cumenesulfonic acid, dodecylbenzenesulfonic acid, pentadecylbenzenesulfonic acid and dinonylnaphthalenesulfonic acid.

The invention involving in claim 4 is a process for inhibiting polymerization of an aromatic vinyl compound which is characterized by adding a quinone methide compound represented by the above-described general formula (1) together with a sulfonic acid compound represented by the above-described general formula (2) to the aromatic vinyl compound in the stage of producing, purifying, storing or transporting the aromatic vinyl compound.

The invention involving in claim 5 is the process for inhibiting polymerization of an aromatic vinyl compound as claimed in claim 4 wherein a N-oxyl compound represented by the following general formula (3) is further added simultaneously 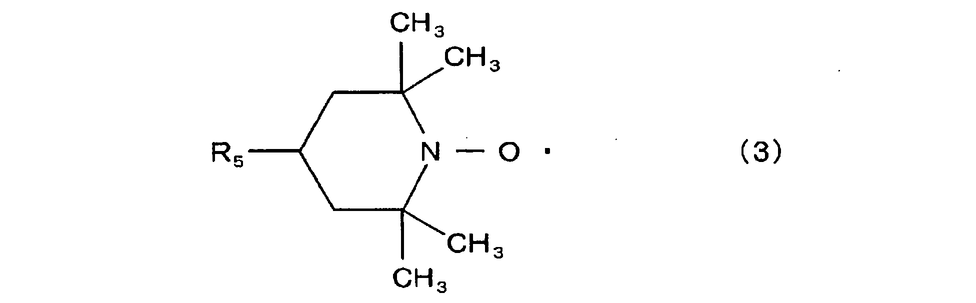 wherein R₅ represents a hydrogen atom, an oxygen atom, a hydroxyl group, an alkyl group having 1-3 carbon atoms, an alkoxyl group having 1-3 carbon atoms, a carboxylic acid having 1-3 carbon atoms or an amide having 1-3 carbon atoms.

The invention involving in claim 6 is the process for inhibiting polymerization of an aromatic vinyl compound as claimed in claim 5 wherein said N-oxyl compound is at least one kind of 2,2,6,6-tetramethylpiperidine-1-oxyl, 4-hydroxy-2, 2,6,6-tetramethylpiperidine-1-oxyl and 4-oxo-2,2,6,6-tetramethylpiperidine-1-oxyl.

The present invention relates to a polymerization inhibitor for an aromatic vinyl compound and to a process for inhibiting polymerization of the aromatic vinyl compound in the stage of producing, purifying, storing or transporting the aromatic vinyl compound, the target aromatic vinyl compound being styrene, methylstyrene, ethylstyrene, divinylbenzene and the like.

The present invention is a polymerization inhibitor for an aromatic vinyl compound which contains a quinone methide compound and an organic sulfonic acid compound, and which is effective for preventing the polymerization of the aromatic vinyl compound by adding them to the stage involving in the above-mentioned aromatic vinyl compound.

The quinone methide compound for use in the present invention is represented by the above-described general formula (1) wherein each of R₁ and R₂ is independently an alkyl group having 4-18 carbon atoms, a cycloalkyl group having 5-12 carbon atoms or a phenylalkyl group having 7-15 carbon atoms, and R₃ is a phenyl group or a phenyl group having 1 to 3 substituent groups, said substituent group being selected from an alkyl group having 1-8 carbon atoms, an alkoxyl group having 1-8 carbon atoms, an alkylthio group having 1-8 carbon atoms, an alkoxycarbonyl group having 2-8 carbon atoms, a hydroxyl group, a nitro group, a cyano group, a carboxyl group, a carbamoyl group and chlorine atom. Specifically, 2,6-di-tert-butyl-4-benzylidene-cyclohexa-2, 5-dienone, 2,6-di-tert-butyl-4-(4-nitrobenzylidene)-cyclohexa-2,5-dienone, 2,6-di-tert-butyl-3-(4-nitrobenzylidene)-cyclohexa-2,5-dienone, 2,6-di-tert-butyl-4-(4-cyanobenzylidene)-cyclohexa-2,5-dienone, 2,6-di-tert-butyl-4-(4-methoxybenzylidene)-cyclohexa-2,5-dienone and 2,6-di-tert-butyl-4-(3,5-di-tert-butyl-4-hydroxybenzylidene)-cyclohexa-2,5-dienone and the like may be taken. Among them, 2,6-di-tert- butyl-4-benzylidene-cyclohexa-2, 5-dienone is especially preferred. These quinone methide compounds may be used singly or in combination of two or more kinds.

They may be obtained by reacting 2,6-di-tert-butylphenol with benzaldehyde having the corresponding substituent group(s).

The organic sulfonic acid compound for use in the present is organic series of free sulfonic acid compound represented by the general formula (2). The alkali metal salts such as those of sodium, potassium and the like, the alkaline earth metal salt such as calcium, the amine salt and other salt which is in the form of complex are excluded because they do not appreciably exert the intended effect of the present invention. In the formula, R₄ is a straight or branched chain alkyl group having 1-32 carbon atoms, an alkylphenyl group in which the alkyl goup is a straight or branched chain one having 1-32 carbon atoms or an alkylnaphthyl group in which the alkyl group is a straight or branched chain one having 1-32 carbon atoms. Specifically, methanesulfonic acid, toluenesulfonic acid, xylenesulfonic acid, cumenesulfonic acid, dodecylbenzenesulfonic acid, pentadecylbenzenesulfonic acid, dinonylnaphthalenesulfonic acid and the like may be taken. These organic sulphonic acid compounds may be used singly or in combination of two or more kinds.

The N-oxyl compound which may be employed in addition to the quinone methide compound and the organic sulfonic acid compound in the stage of producing, purifying, storing or transporting the aromatic vinyl compound is represented by the general formula (3) and is steric hindered N-oxyl compound having steric hindered substituent group(s) linked adjacently to N-oxyl radical. In the formula, R₅ represents a hydrogen atom, an oxygen atom, a hydroxyl group, an alkyl group having 1-3 carbon atoms, an alkoxyl group having 1-3 carbon atoms, a carboxylic acid group having 1-3 carbon atoms or an amide group having 1-3 carbon atoms. As the alkyl group having 1-3 carbon atoms, methyl, ethyl, propyl and isopropyl groups may be taken. As the alkoxyl group having 1-3 carbon atoms, methoxy, ethoxy, propoxy and isopropoxy groups may be taken. As the carboxylic acid group having 1-3 carbon atoms, formic acid, acetic acid and propionic acid groups may be taken. As the amide group having 1-3 carbon atoms, formic acid amide group, acetic acid amide group, propionic acid amide group and the like may be taken. Specifically, 2,2,6,6-tetramethylpiperidine-1-oxyl, 4-hydroxy-2,2,6,6-tetramethylpiperidine-1-oxy, 4-oxo-2, 2,6,6- tetramethylpiperidine-1-oxyl, 4-methoxy-2, 2,6,6-tetramethylpiperidine-1-oxyl, 4-ethoxy-2, 2,6,6-tetramethylpiperidine -1-oxyl, 4--carboxyl-2,2,6,6-tetramethylpiperidine-1-oxyl, 4-carbamoyl-2,2,6,6-tetramethylpiperidine-1-oxyl and the like may be taken. Are preferred 2,2,6,6-tetramethylpiperidine-1-oxyl, 4-hydroxy-2, 2,6,6-tetramethylpiperidine-1-oxyl and 4-oxo-2,2,6,6-tetramethylpiperidine-1-oxyl. These N-oxyl compounds may be used singly or in combination of two or more kinds.

The polymerization inhibitor of the present invention is one comprising two ingredients of the quinone methide compound and the organic sulfonic acid compound, and it may be usually used in the form dissolved in an aromatic organic solvent. As specific aromatic organic solvent, benzene, toluene, xylene, ethylbenzene and the like may be taken. One or more of them may be used.

Each concentration of the quinone methide compound and the organic sulfonic acid compound in the polymerization inhibitor of the present invention cannot be determined as a rule and may be adequately determined. However, it may be generally 1-20 % by weight. Also, in order to improve the solubility and the solution stability of the quinone methide compound, alcohols (e.g. ethylene glycol, butyl glycol and the like) may be incorporated with no problem. The polymerization inhibitors which have been used hitherto may be further incorporated to such an extent that the effect of the present invention is not injured.

As to the mixture ratio of the respective ingredients in the polymerization inhibitor of the present invention comprising two ingredients of the quinone methide compound and the organic sulfonic acid compound, it cannot be determined as a rule depending on the kind and amount of the intended aromatic vinyl compound, the operation status and the extent required for the polymerization-inhibitory effect and it may be adequately determined. However, the mixture ratio of the quinone methide compound to the organic sulfonic acid compound is usually 95:5 - 5:95 (by weight ratio), preferably 90:10-10:90, more preferably 80:20-20:80.

The polymerization inhibitor is basically classified into the polymerization-suppressing type which retards rate of polymerization and into the polymerization-preventing type which retards the initiation time of the polymerization. Although the actual polymerization inhibitor cannot be strictly divided into either of these two types, the quinone methide compound and the organic sulfonic acid compound in the present invention have high tendency to be the polymerization-suppressing type while the N-oxyl compound is the polymerization-preventing type. Although the polymerization-suppressing type is inferior in the initial stage of polymerization inhibition, it is capable of inhibiting the polymerization over relatively long period of time. On the other hand, although the polymerization-preventing type is excellent in the initial stage of polymerization inhibition, it is consumed by the reaction with the radical present in the system with the lapse of certain time and thereafter the polymerization-inhibitory effect is substantially lost. Accordingly, it is desirable to set the optimum mixture ratio of the both polymerization inhibitors to be added depending on the extent required for the stage. For example, in the case where it is desired to inhibit the polymerization for a long time in considering the status of the stage, the ratio occupying the polymerization-suppressing type is increased while in the case where it is desired to prevent the initial stage of polymerization completely, the ratio occupying the polymerization-preventing type is increased.

The preparation process of the polymerization inhibitor involving in the present invention is not restricted particularly. The polymerization inhibitor may be obtained by dissolving the quinone methide compound and the organic sulfonic acid compound in an aromatic organic solvent uniformly under stirring.

In the polymerization inhibitor of the present invention, the quinone methide compound is used together with the organic sulfonic acid compound whereby the generation of radical may be inhibited by the organic sulfonic acid compound and the chain-extension of radical may be inhibited by the quinone methide compound so that the radical polymerization may be inhibited and the synergic effect based on their combination may be obtained. Particularly, as the temperature becomes higher, the synergic effect tends to increase.

In the stage treating the aromatic vinyl compound, there exists often the place where its retention time is long, and hence in general the polymerization-suppressing type is preferably selected. However, it may be effectively used in the case that the polymerization-preventing function is intended to be further imparted depending on the status of the stage and for this purpose it is recommended that N-oxyl compound of the polymerization-preventing type is further added.

The polymerization inhibition process of the present invention is a process for inhibiting the polymerization of said aromatic vinyl compound and is also a process for preventing fouling resulted from the polymerization of the aromatic vinyl compound by adding the above-described quinone methide compound and organic sulfonic acid compound or by adding the quinone methide compound, organic sulfonic acid compound and N-oxyl compound to the stage of producing, purifying, storing or transporting the aromatic vinyl compound.

The amount added of the polymerization inhibitor involving in the present invention to the stage of producing, purifying, storing or transporting the aromatic vinyl compound can not be determined as a rule but it is to be determined in considering the condition, the extent required, etc. of the stage to be added. The amount added of quinone methide compound is usually 10-2000 ppm, preferably 100-1000 ppm based on the aromatic vinyl compound, that of organic sulfonic acid compound is usually 10-1000 ppm, preferably 50-500 ppm based on the aromatic vinyl compound, and that of N-oxyl compound is usually 0.5-1000 ppm, preferably 5-100 ppm based on the aromatic vinyl compound. These suitable ranges are the standard amount to be added. When it is smaller than this range, the effect of the present invention is not significant in some case. When the respective amounts of quinone methide compound and N-oxyl compound are greater than the above-described range, the polymerization-inhibitory effect becomes higher but in some cases the elevation in the polymerization-inhibitory effect is little relative to the increase in the added amount and hence the cost performance is lowered. When the amount added of organic sulfonic acid compound is greater than the above-described range, not only it brings about an economical disadvantage but also it induces an ionic polymerization of the aromatic vinyl compound in some case.

As the polymerization inhibition process of the present invention, there are one wherein a polymerization inhibitor containing the two ingredient of quinone methide compound and organic sulfonic acid compound is added with a pump, one wherein the polymerization inhibitor containing the two ingredient of quinone methide compound and organic sulfonic acid compound is added while N-oxyl compound is separately added thereby these three ingredients are allowed to be acted together at the place causing a problem, one wherein quinone methide compound, organic sulfonic acid compound and N-oxyl compound are simultaneously added, one wherein quinone methide compound and organic sulfonic acid compound are separately added thereby these two ingredients are allowed to be acted together at the place causing a problem, one wherein quinone methide compound, organic sulfonic acid compound and N-oxyl compound are individually added thereby these three ingredients are allowed to be acted together at the place causing a problem, and the like one. Thus the intended one may be selected depending on the operation condition and the extent of problem of process, the extent required for improving the problem and from the economical aspect. For example, for the purpose of inhibiting the polymerization for a long time, the combination of quinone methide compound and organic sulfonic acid is recommended while for the purpose of preventing the initial stage of polymerization completely, the combination of additional N-oxyl compound with them is recommended.

The addition means of the polymerization inhibitor involving in the present invention is not restricted particularly. However, the polymerization inhibitor is usually added with an infusion pump for chemicals. Although the place to which the polymerization inhibitor involving in the present invention is added is not particularly restricted, the polymerization inhibitor is usually added to the upstream process over the place at which aromatic vinyl compound undergoes polymerization to cause the problem as fouling. For example, styrene is generally manufactured by dehydrogenation reaction of ethyl benzene, and the formed styrene is continuously separated from the unreacted ethyl benzene by distillation, and therefore it is recommendable that the polymerization inhibitor is supplied to the distillation columns after the dehydrogenation reaction of ethyl benzene has been conducted. As to the place to which the polymerization inhibitor is added, such an application is adequately selected as in that the polymerization inhibitor is added to the specific place at one stroke or otherwise it is added to some places in portions.

### The Best Mode For Carrying Out The Invention

The present invention is illustrated in more details by the working example but it is not restricted to the following working examples.

### [Compounds used for evaluation]

### (Quinone methide compound)

### A-1: 2,6-di-tert-butyl-4-benzylidene-cyclohexa-2,5-dienone

2,6-Di-tert-butyl-4-benzylidene-cyclohexa-2,5-dienone was synthesized according to the method described in "Synth.Commun." (B. Koutek et al.; 6 (4), 305 (1976)]. 2.1 Grams (10 mmol) of 2,6-di-tert-butyl phenol, 1.06 g (10 mmol) of benzaldehyde, 0.9 g (10.5 mmol of piperidine and 15 ml of n-butanol were placed in a 100 ml of eggplant type flask equipped with a Dimroth condenser and refluxed for 24 hours under an atmosphere of nitrogen. After the reflux, n-butanol was distilled out under reduced pressure and the reaction product was purified by silica gel column chromatography (a development solvent of n-hexane:methylene chloride=2:1) to obtain quinone methide compound (A-1).

### A-2: 2,6-di-tert-butyl-4-(4-nitrobenzylidene)-cyclohexa-2,5-dienone

Quinone methide compound (A-2) was obtained by a similar manner as in quinone methide compound (A-1) except that 1.51 g of 4-nitrobenzaldehyde was used in place of benzaldehyde.

### A-3: 2,6-di-tert-butyl-4-(4-cyanobenzylidene)-cyclohexa-2,5-dienone

Quinone methide compound (A-3) was obtained by a similar manner as in quinone methide compound (A-1) except that 1.31 g of 4-cyanobenzaldehyde was used in place of benzaldehyde.

### [Sulphonic acid compound]

- B-1:: dodecylbenzenesulfonic acid (reagent class, a product of Tokyo Kasei Kogyo Co., Ltd.)
- B-2:: pentadecylbenzenesulfonic acid ("Taycacure AC-330" (a trade name, a product of TAYCA Corporation)

### [N-Oxyl compound]

- C-1:: 4-hydroxy-2,2,6,6-tetramethylpiperidine-1-oxyl (reagent class, a product of Aldrich Co., Ltd.)
- C-2:: 4-oxo-2,2,6,6-tetramethylpiperidine-1-oxyl (reagent class, a product of Aldrich Co., Ltd.)

### [Other]

- DNBP:: 2,4 dinitro-6-sec-butyl phenol

### [Polymerization-inhibiting test]

100 Grams of styrene monomer (before the beginning of the experiment, any polymerization inhibitors contained in the monomer had been removed by an alkali washing and then the resultant monomer had been washed with water and dried) was placed in a four-necked separable flask equipped with a reflux condenser and the prescribed amount of the polymerization inhibitor was added thereto, and highly purified nitrogen gas was introduced at the quantity of flow of 80 ml/ min. while the temperature was kept to 120 °C. A part of the content therein was taken out every time given and 9 times volume of methanol based on the content taken out was added thereto to precipitate the formed polymer in a suspended state, which was filtrated out to weigh its weight amount (g) and the polymer concentration (wt. %) in the monomer was evaluated. The results are summarized in table 1.

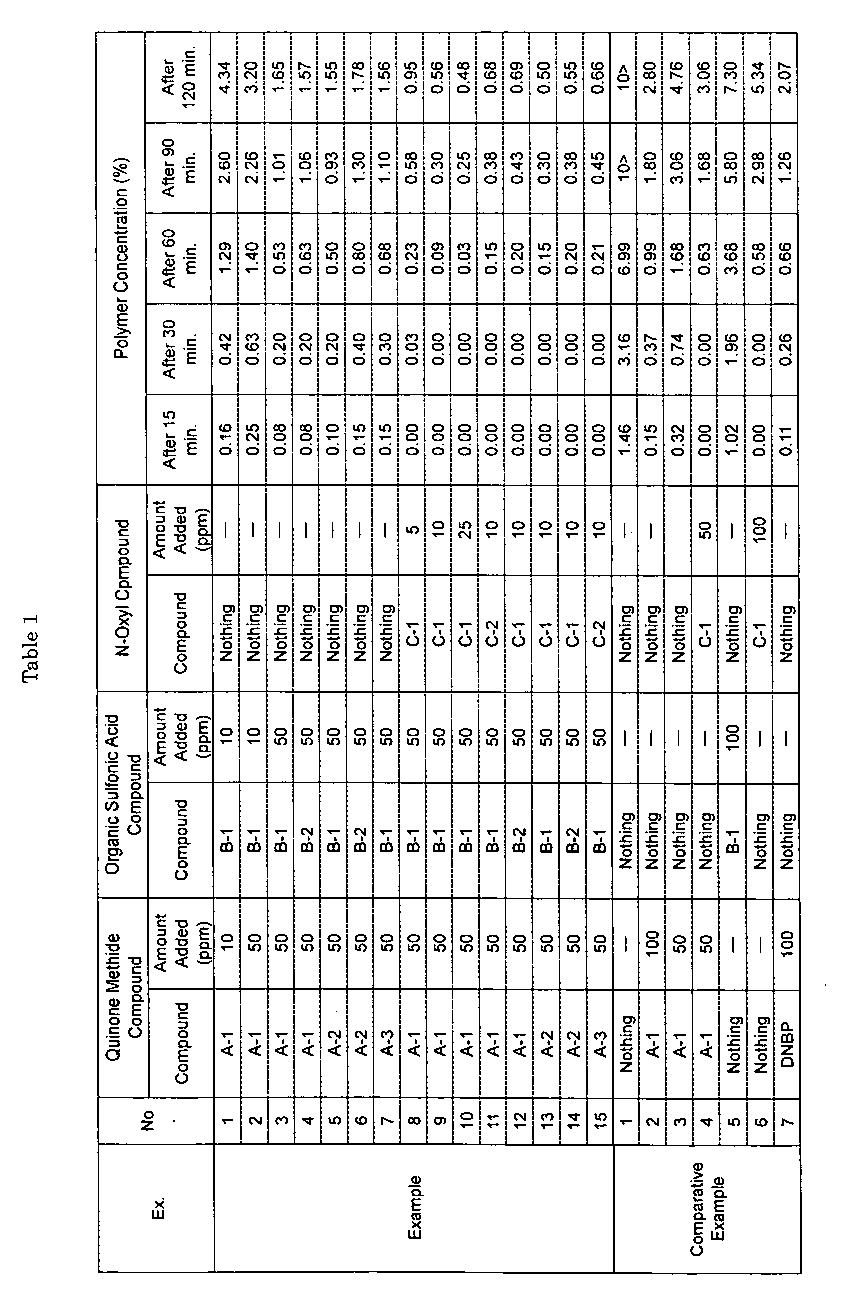

It became clear that the polymerization inhibitor containing two ingredients of a quinone methide compound and an organic sulfonic acid compound involving in the present invention (Example Nos. 1-7) shows not only superior effect to 2,4-dinitro-6-sec-butyl phenol (DNBP) of Comparative Example No.7 which had been generally used in the distillation and purification stages of styrene but also higher effect than quinone methide compound alone (Comparative Example No.2).

Also, it was recognized that in the case where the polymerization inhibition was conducted using three ingredients of quinone methide compound, organic sulfonic acid compound and N-oxyl compound involving in the present invention (Example Nos. 8-15), the incorporation of a small amount of N-oxyl compound caused an effect to prolong the initial term in the polymerization inhibition caused by the polymerization inhibitor containing two ingredients of quinone methide compound and organic sulfonic acid compound (Example Nos. 1-7) thereby the polymerization may be inhibited over a long term.

### Industrial applicability

By the polymerization inhibitor and the polymerization inhibition process of the present invention the polymerization including the initial polymerization may be inhibited over a long term in the stage of producing, purifying, storing or transporting it. This makes it possible to inhibit efficiently the generation of soil (fouling) in these stages thereby operating the stages stably for a long period of time and attributing greatly to the stabilization of the stages and further to the elevation in the safety operation of the stages.

## Claims

1. A polymerization inhibitor for an aromatic vinyl compound which is **characterized by** containing a quinone methide compound represented by the following general formula (1) together with an organic sulfonic acid compound represented by the following general formula (2) 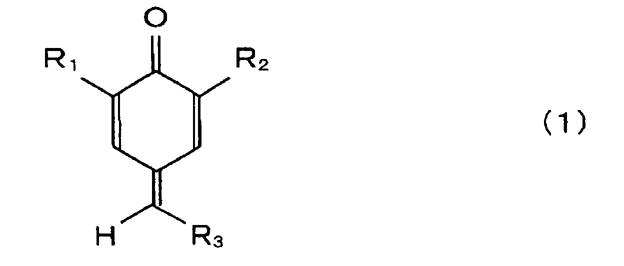 wherein R 1 and R 2 represent independently an alkyl group having 4-12 carbon atoms, a cycloalkyl group having 5-12 carbon atoms or a phenylalkyl group having 7-15 carbon atoms, and R₃ represents a phenyl group or a phenyl group having 1 to 3 substituent groups, said substituent group(s) being selected from an alkyl group having 1-8 carbon atoms, an alkoxyl group having 1-8 carbon atoms, an alkylthio group having 1-8 carbon atoms, an alkoxycarbonyl group having 2-8 carbon atoms, a hydroxyl group, a nitro group, a cyano group, a carboxyl group, a carbamoyl group and chlorine atom. 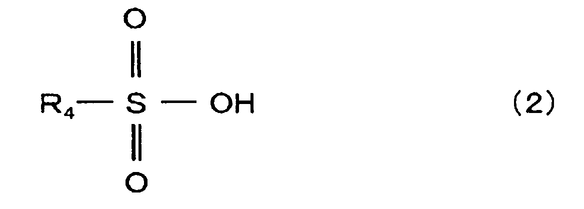 wherein R₄ represents a straight or branched chain alkyl group having 1-32 carbon atoms, an alkylphenyl group wherein the alkyl group is a straight or branched chain one having 1-32 carbon atoms or an alkylnaphthyl group wherein the alkyl group is a straight or branched chain one having 1-32 carbon atoms.

2. The polymerization inhibitor for an aromatic vinyl compound as claimed in claim 1 wherein said quinone methide compound is one or more members selected from 2,6-di-tert-butyl-4-benzylidene-cyclohexa-2, 5-dienone, 2,6-di-tert-butyl-4-(4-nitrobenzylidene)-cyclohexa-2, 5-dienone, 2,6-di-tert-butyl-3-(4-nitrobenzylidene)-cyclohexa-2, 5-dienone, 2,6-di-tert-butyl-4-(4-cyanobenzylidene)-cyclohexa-2, 5-dienone, 2,6-di-tert-butyl-4-(4-methoxybenzylidene)-cyclohexa-2, 5-dienone and 2,6-di-tert-butyl-4-(3,5-di-tert-butyl-4-hydroxybenzylidene)-cyclohexa-2, 5-dienone.

3. The polymerization inhibitor for an aromatic vinyl compound as claimed in claim 1 wherein said organic sulfonic acid compound is one or more members selected from methanesulfonic acid, toluenesulfonic acid, xylenesulfonic acid, cumenesulfonic acid, dodecylbenzenesulfonic acid, pentadecylbenzenesulfonic acid and dinonylnaphthalenesulfonic acid.

4. A process for inhibiting polymerization of an aromatic vinyl compound which is **characterized by** adding a quinone methide compound represented by the above-described general formula (1) together with a sulfonic acid compound represented by the above-described general formula (2) to said aromatic vinyl compound during the stage of producing, purifying, storing or transporting the aromatic vinyl compound.

5. The process for inhibiting polymerization of an aromatic vinyl compound as claimed in claim 4 wherein a N-oxyl compound represented by the following general formula (3) is further added simultaneously 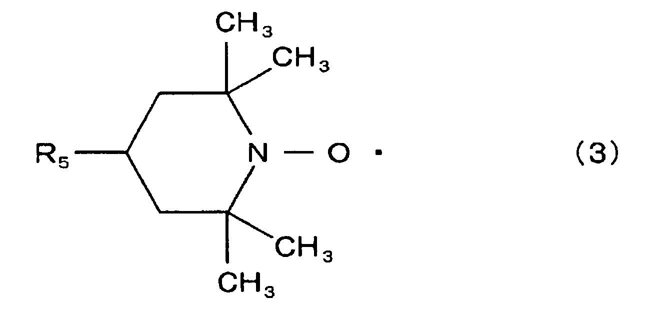 wherein R₅ represents a hydrogen atom, an oxygen atom, a hydroxyl group, an alkyl group having 1-3 carbon atoms, an alkoxyl group having 1-3 carbon atoms, a carboxylic acid having 1-3 carbon atoms or an amide having 1-3 carbon atoms.

6. The process for inhibiting polymerization of an aromatic vinyl compound as claimed in claim 5 wherein said N-oxyl compound is at least one of 2,2,6,6-tetramethylpiperidine-1-oxyl, 4-hydroxy-2, 2,6,6-tetramethylpiperidine-1-oxyl and 4-oxo-2, 2,6,6-tetramethylpiperidine-1-oxyl.
